# EUROPEAN PATENT APPLICATION

(11) **EP 2 452 720 A1**
(43) Date of publication of application: **16.05.2012**
(21) Application number: 11184959.2
(22) Date of filing: 13.10.2011
(51) Int. Cl.: A61M 39/10

(54) **Non-drip coupling device for transferring a fluid**

(30) Priority: 13.10.2010 DK 201000933
(71) Applicant: CleanConnect ApS, 6430 Nordborg (DK)
(72) Inventor: Bendt, Mads, DK-8310 Tranbjerg J (DK); Voss, Fands Wulff, DK-6400 Sonderborg (DK)
(74) Representative: Inspicos A/S

(57) **Abstract**

The new invention concerns a non-drip fluid coupling device intended for transferring a flow of at least one substance e. g. a fluid, where the fluid coupler comprises a first coupler and a second coupler, where
- the first coupler has first chamber connected to a first fluid communication and an first opening, a first valve body being biased against the first opening by a first biasing member,
- the second coupler has a second chamber connected to a second fluid communication and a second opening, a second valve body biased against the second opening by a second biasing member,
- a hollow needle element is positioned or formed within the second chamber and comprising a sealing element,

wherein the first and second coupler comprises guiding means adapted to aid the connection of the first and second couplers.

## Description

### Technical field

The new invention concerns a non-drip fluid coupling device intended for transferring a flow of at least one substance e. g. a fluid.

### Background and purpose of the invention

Many reasons exist for wanting a fluid coupling to connect flow systems, where no fluid is spilled during coupling and decoupling of the systems, such as for safety or comfort.

One example is the comfort of people equipped with a catheter and a urine bag, where the bag is to be replaced when filled. A comfortable replacement would be easy, and involve no spill of urine during the replacement.

Another reason is, for example, when the fluids are aggressive (e. g. toxic or corrosive). Then it is critical to ensure that no dripping or depositing of fluids occurs during connection and disconnection of e.g. fluid reservoirs to an operation system or fuel cells.

In the following, the main embodiment example is the introduction of a non-drip fluid connector for the catheter / urine bag coupling, but any other use where a non-drip fluid connector would be appreciated would also apply to the present invention.

### Prior art

A number of documents concern such fluid couplings. US 5,293,902 describes a quick disconnect coupling for connection to a fluid fitting, having a Schroeder valve, said coupling comprising:
- a body formed with an axial bore and a passageway in communication with said bore, said bore being formed with an open end to receive said fluid fitting there-within and an annular shoulder ;
- an annular seal within said bore mounted in a groove formed in said body for sealingly engaging an outside diameter of said fluid fitting upon receipt of said fluid fitting;
- a locking means for removably attaching said body to said fluid fitting and for maintaining contact of said annular seal with a ridge portion of said fluid fitting said locking means being reciprocally attached to said body;
- a single valve pin reciprocally mounted within said axial bore for movement between a closed position wherein said valve pin engages said annular shoulder of said bore to create a seal and prevent communication between said open end and of said passageway, and an open position wherein communication is permitted, said valve pin being moveable in response to engagement with said fluid fitting, said valve pin including an extension for contacting and opening the Schroeder valve and a base portion for contacting said fluid fitting and for limiting penetration of said extension into said fluid fitting; and
- a biasing means connected to said body and said valve pin for adjustably biasing said valve pin into said closed position.

This system has the disadvantage that it is not a non-dripping coupling since the axial bore (16) would contain fluid when disconnected and a sealing of the fluid fitting (54) does not exist.

WO 2006/043883 describes a coupling device intended for transferring a flow of a substance, which device comprises a first coupling element and a second coupling element. The first coupling element comprises a fluid channel and a first sealing element displaceable from a sealing position under the effect of spring force, and also a rigid first displacing element. The second coupling element comprises a fluid channel and a second sealing element displaceable from a sealing position under the effect of spring force and also a rigid second displacing element. Said sealing elements on each coupling element are arranged to sealingly close the inlet (outlet) of the fluid channel when the two coupling elements are separated. The first displacing element is arranged to displace the second sealing element out of the sealing position when the said coupling elements are connected and the second displacing element is arranged to displace the first sealing element on the first coupling element out of the sealing position when the said coupling occurs. The two coupling elements have smooth coupling surfaces, which are separated from the flow through the fluid channels after connecting the coupling elements.

The couplings in both documents, however, have several pieces to be fitted into bores complicating the manufacturing process and increasing the risk of malfunctions of the parts in the bores which might lead to a leaky connection during operation. Further they have special shapes adapted to fit together which complicates the manufacturing processes as well.

For connectors such as for urine bags it is essential that the connector is cheap to manufacture and at the same time is connected in a stable manner with no risk of the connector decoupling during operation of the system using the connector.

### Object of the invention

It is an object of this invention to design a stable non-dripping fluid coupling system without the drawbacks of the prior art and which is able to form a secure fluid coupling where it is easy to connect the couplers, and where the coupling system is simple and cheap to manufacture.

This is solved by introducing a non-drip fluid coupler comprising a first coupler and a second coupler, where
- the first coupler has first chamber connected to a first fluid communication and a first opening, a first valve body being biased against the first opening by a first biasing member,
- the second coupler has a second chamber connected to a second fluid communication and a second opening, a second valve body being biased against the second opening by a second biasing member,
- a hollow needle element is positioned or formed within the second chamber and comprises a sealing element-,
wherein the first and second coupler comprise guiding means adapted to aid the connection of the first and second couplers.

These guiding means helps to connect the first and second couplers.

In one embodiment the guiding means comprises
- first deepening(s) of one of the couplers, where the cross section area of the first deepening(s) is substantially funnel shaped having an outer section and an inner section, where the outer width of the outer section is substantially larger than any width of the inner section, and
- first protruding structure(s) with a shape fitting into the inner section(s).

The fluid couplers further comprise interlocking means preventing them from any movement in a longitudinal direction when coupled, this ensuring a stable connection preventing unintended decoupling.

In one embodiment the interlocking means comprises rotation guiding means assisting to couple them by rotation, where in a more specific embodiment the rotation guiding means comprises at least one 'receiving' shape of one of the couplers and at least one wing of the other coupler adapted to fit into the receiving' shape(s,) and where the wing(s) are introduced into the receiving shape(s) by rotating the couplers relative to each other.

Since, in one embodiment, the coupling is in a rotation, the combined first protruding structure(s) may be rotationally symmetric.

To assist the rotation of the sealing element, one of the first and second valve bodies has second protrusion(s) matching a second deepening of the other of the first and second valve bodies, and in a more specific embodiment the second protrusion and the second deepening are shaped such that when mated they are interlocked in a rotational direction.

The hollow needle element comprises at least one needle opening being sealed by the second valve body when the first and second couplers are not coupled.

To ensure a sealed second coupler even in high pressure systems, a third valve body is introduced to be biased against a narrowing of the hollow needle element and the sealing element by a third biasing member. The contact surfaces between the third valve body and the sealing element is such that a rotation of the sealing element moves the third valve body away from the sealing element, where this In one embodiment is solved by forming the contact surfaces such that they has an angle different to being orthogonal to a longitudinal axis relative the extension of the alignment line of the third valve body and the sealing element.

In an alternative embodiment the contact surfaces are winded, or threaded.

To ensure the rotation of only the sealing element, the first and third valve bodies are prevented from rotating within the first chamber and hollow needle, respectively.

To ensure a fluid tight first coupler when not coupled, the first opening has a narrowing matching the shape of the first valve body in such a manner that, when the first valve body is in the closed position, the first opening is sealed from any fluid communication through the first opening.

### Figures

- Fig. 1: Illustration of the first and second couplers introducing a wing and receiving shape according to one embodiment of the present invention.
- Fig. 2: Illustration of the components of the first and second couplers according to one embodiment of the present invention.
- Fig. 3: Illustration of the first and second couplers according to Fig. 2 when coupled.
- Figs. 4A and 4B: Illustrations of the first and second couplers according to Fig. 2 during coupling / decoupling.
- Figs. 5A and 5B: Illustrations of a second embodiment of the first and second couplers.
- Figs. 6A and 6B: Illustration of valve bodies with slide extensions and one recesses of the valve chamber matching the slide extensions.
- Fig. 7: Illustration of the valve body having two sets of parallel slide extensions.
- Fig. 8: Illustration of valve bodies and corresponding biasing members formed as one single body according to one embodiment of the present invention.

### Detailed description of the invention

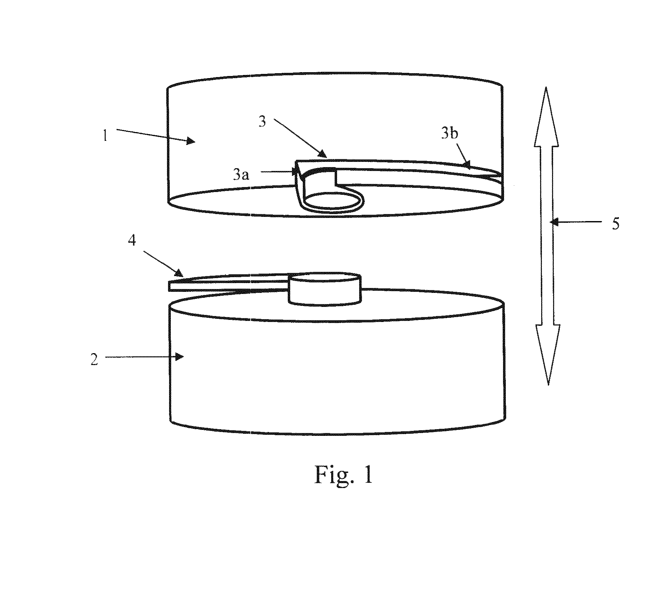

One first aspect of the present invention is to ensure a simply and easily connectable, securely interlocked coupling, therefore means is introduced especially to guide the two parts during coupling and to prevent the couplers (1, 2) from un-inherently decoupling. In one not limiting embodiment this is achieved by forming rotation guiding means (3, 4) on the first (1) and second coupler (2). The rotation guiding means formed is as windings (being threaded), where one of the couplers (1, 2) is screwed into the other, the windings being the rotation guiding means (3, 4). In Fig. 1 rotation guiding means (3) and (4) are introduced as one or more 'wings' (4) at the second coupler (2) and 'receiving' shape(s) (3) of the first coupler (1) (or vice versa). When the second coupler (2) and first coupler (1) are connected, the wing(s) (4) is positioned in the introducing part(s) (3a) of the 'receiving' shape(s) (3), and when rotated the wing (4) is guided into a 'locking' section (3b) of the 'receiving' shape. In this position the first (1) and second (2) couplers are locked and prevented from being detached in a longitudinal direction (5).

Any other alternative of interlocking as known in the art of interlocking such two couplers would also apply to the present invention.

Though the illustration shows rotation guiding means (3) and (4) to form the interlocking means to prevent the couplers from un-intended decoupling, especially in the longitudinal direction (5), other kinds of interlocking means as known in the art would also apply to the present invention.

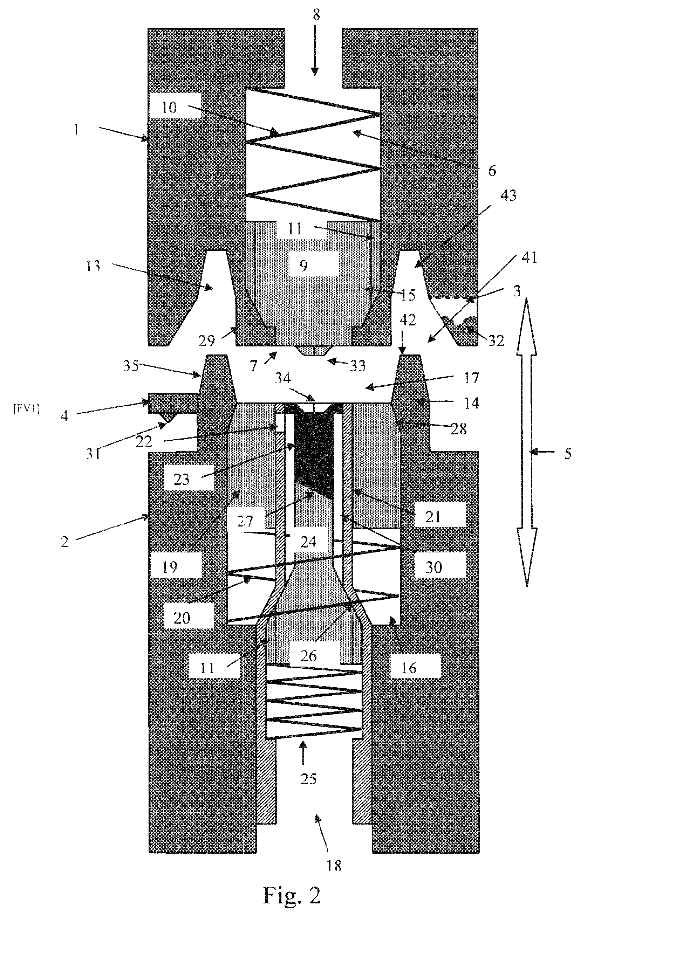

Fig. 2 illustrates schematically the first (1) and second (2) couplers. The first coupler (1) has a first chamber (6) with a first opening (7), a connection to a first fluid communication (8), a first valve body (9) being biased against the first opening (7) by first biasing member (10). The first valve body (9) in one embodiment is prevented from rotation within the first chamber (6), where in the example illustrated, the first valve body (9) is equipped with slide extensions (11) being fixated by structures (or rifts) inside the first chamber (6), these structures not illustrated. Only it has to be ensured that the first valve body (9) does not seal off flow within the first chamber (6) itself, only in the area of the first opening (7). The biasing member (10) could optionally be a spring inserted into the first chamber (5) being squeezed between the first valve body (9) and the internal wall of the first chamber (6).

In the illustrated embodiment in Fig. 2, the first coupling (1) further comprises first deepening(s) (13) and the second coupler (14) comprises first protrusion(s) (14).Vice versa would also apply. The first protrusion(s) (14) preferably comprises extended protrusion part(s) (35) optionally formed with at least one sloping side, such that the extended protrusion part(s) (35) are narrowing towards the front surface(s). The first deepening(s) (13) are formed in a 'funnel' like manner, where they, at their opening, are tapered or widened in a manner such that they assist guiding the first protrusion(s) (14) into the first deepening(s) (13) during coupling. Therefore the opening(s) (41) of first deepening(s) (13) have area(s) substantially larger than the area(s) of the front surface(s) (42) of the first protrusion(s) (14), at least 1.2 times the area, or at least 1.5 the area or at least two times the area or at least five times.

The first deepening(s) (13) and first protrusion(s) (14) are positioned and shaped such that they mate when the couplers are connected, and where the substantially enlarged opening area (41) of the first deepening(s) (13) relative to the area of the front surface (42) helps the first protrusion(s) to 'find' the first deepening(s) when the couplers are connected, thus working in a funnel like manner. The 'inner' section (43) of the first deepening(s) (41) is preferably shaped to match the first protrusion(s) (14) more or less tightly.

The first protrusion(s), especially in the embodiment where the couplers (1) and (2) are coupled by a rotation, the first protrusion(s) (14) would. at least in combination, be preferred to be rotation symmetric, and the first deepening(s) (13) would be formed accordingly as needed.

A first narrowing shape (15) forms the transition from the first chamber (6) to the first opening (7), where this first narrowing shape (15) matches the shape of the first valve body (9) in such a manner that, when biased towards the first opening (7) and first narrowing shape (15), the opening is sealed leaving no fluid communication. However, when the first valve body (9) is pushed into the first chamber (6) squeezing the first biasing member (10), the first opening (7) is opened to form a fluid connection from the externals to the first chamber (6) and thus the first fluid communication (8)

The second coupler (2) comprises the second chamber (16) with second opening (17) and is connected to a second fluid communication (18). Inside the second chamber (16) is a second valve body (19) biased against the second opening (17) by a second biasing member (20), a hollow needle element (21) having at least one needle opening (22) forming a fluid communication between the inside of the hollow needle (21) and the second chamber (16), or at least the second fluid communication (18). In the vicinity of the second opening (17), the hollow needle element (21) is fixed within the second chamber (16), and is optionally formed as an integral part of a body forming the second coupler (2), or more preferably as a hollow body, such as a tube shaped body, being positioned within the second chamber (16)).

In the illustrated embodiment, the first opening (7) has an opening matching the hollow needle element (21) in such a manner that it forms a substantially tight sealing to the fluids around the hollow needle element (21) during coupling. Optionally, as illustrated, could be by introducing a sealing element in the first opening (7) of a substantial soft material as they are well known in the arts used as sealing elements.

Inside of the hollow needle (21) is a sealing element (23), which is optionally reaching out of the hollow needle (21). The sealing element (23) seals any fluid access between the externals and the inside of the hollow needle (21). The sealing element (23) is secured to the hollow needle (21) in such a manner that it is only free to rotate around an axis parallel to the longitudinal axis (5), being parallel to the extension of the hollow needle (21), but prevented from moving in any direction parallel to the longitudinal axis (5).

An optional third valve body (24) is biased against the sealing element (23) and a narrowing (26) of the hollow inside the hollow needle (21) by a third biasing member (25). The third valve body is shaped such that when biased against the narrowing (26), the third valve body (24) seals any fluid communication from the second fluid communication (17) through the needle openings (22)

The contact surfaces (27) between the third valve body (24) and the sealing element (23) are such that a rotation of the sealing element (23) around the longitudinal axis (5) forces the third valve body (24) away from the narrowing (26) opening for fluid communication from the second fluid communication (17) to the needle opening(s) (22). In the illustrated embodiment in Fig. 2, the contact surfaces (27) are such that they form a plane having an angle relative to the longitudinal axis (5).

Alternatively the contact surfaces (27) are winded, or threaded) such that the third valve body (24) is screwed in and out of the sealing element (23) or vice versa.

In the same manner as the first valve body (9), the third valve body (24) is prevented from rotation within the second chamber (16), for example by equipping it with slide extensions (11) fixated by structures inside the second chamber (16), these structures not illustrated. Only it has to be ensured that the third valve body (24) does not seal off flow within the hollow needle (21) in general, only in the area of the narrowing (26).

The second chamber optionally has a third narrowing (28) matching an also optional shape of the second valve body (19) in such a manner that, when the second valve body (19) is biased against the second opening (17), it seals off any fluid communication through the second opening (17).

The second valve body (19) is such that when in closed position, it is positioned where the needle openings (22) are situated, thus sealing any fluid communication through the needle openings (22). In one preferred embodiment the second valve body (19) is ring shaped encircling at least the part of the hollow needle (21), where the needle openings (22) are situated.

The second valve body (19) is optionally free to rotate around the longitudinal axis (5) and thereby also the hollow needle (21).

The third valve body (24) and the sealing element (23) are such that, in the section of the hollow needle (21) from the narrow end of the narrowing (26) to the needle opening(s) (22), the internal volume of the hollow needle (21) is not completely filled by the valve body (24) and sealing element (23), thus leaving free channel(s) (30).

Second protrusions (29) are formed between the first deepenings (13) and the first opening (7), preferable encircling the first opening (7).

The first valve body (9) is seen to have third protrusions (33) matching third deepenings (34) of the sealing element (23) (or vice versa) in a manner where, when mated, they are interlocked in a rotational direction.

The illustrated embodiment is shown having one 'wing' (4) having optionally at least one fourth protrusion (31) matching fourth deepening(s) (32) of the receiving shape (3). These are formed such that, when coupled, the biasing members such as (10, 20, 25), will attempt to push the first (1) and second (2) couplers apart in the longitudinal direction (5), and will therefore also push the fourth protrusion(s) (31) into the fourth deepening(s) (32) in a manner that, the further rotation is prevented until a force against the biasing members push the fourth protrusion(s) (31) out of the fourth deepening(s) (32). This helps to keep the couplers (1) and (2) firmly connected during operation.

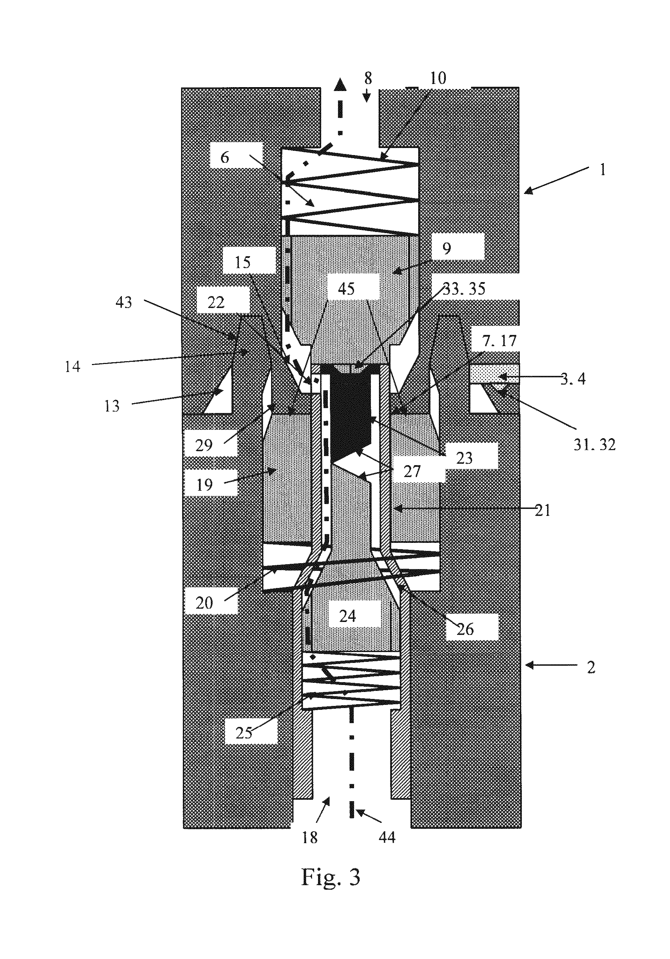

Fig. 3 illustrates the situation where the two couplers (1) and (2) are coupled.

The first protrusions (14) have been introduced into the first deepenings (13) and positioned in the inner section(s) (43), and the 'wing' (4) has been rotated into the 'receiving' shape (3). In the same rotation the first valve body (9) being prevented from rotation within the first chamber (6), has rotated the sealing element (23). This has been helped by the mating deepening (34) and protrusion (33) (much in the same manner as a screwdriver acts upon a screw).

By the contact surfaces (27), the rotation of the sealing element (23) has pushed the third valve body down, freeing it from the narrowing (26) and thus forming fluid connection from the second fluid communication (18) through the inside of the hollow needle (21) to the needle openings (22).

At the same time, the third protrusion (29) has pushed the second valve body (19) away from the needle opening(s) (22) thus forming fluid connection from the needle opening(s) (22) to the first chamber (6).

The needle element (21) has pushed the first valve body (9) into the first chamber (6), thus unsealing the first opening (7) forming a fluid connection from the first (7) to the second (17) opening, and thereby a fluid connection from the second fluid communication (18) to the first fluid communication (8).

The wing (4) is seen to have been rotated into the receiving shape (3), the fourth protrusion (31) being pushed into matching fourth deepening(s) (32) by the biasing members (10, 20, 25).

The arrow (44) illustrates one way of flow of the fluids through the coupling, where the flow could naturally also flow in the other direction.

The part of the hollow needle (21) with the needle openings (22) extends through the first opening (7) and reaches into the first chamber (6).

Especially because the first (9) and third (24) valve bodies are formed to be pressed against the narrowing shapes (15) and (26), respectively, the advantage occurs that pressure from fluids within the first (10) and second chambers (16) when the couplers (1) and (2) are not coupled, will only assist the biasing members (6) and (25) in forming tight seals. This embodiment is therefore especially suited even for systems with substantial pressures.

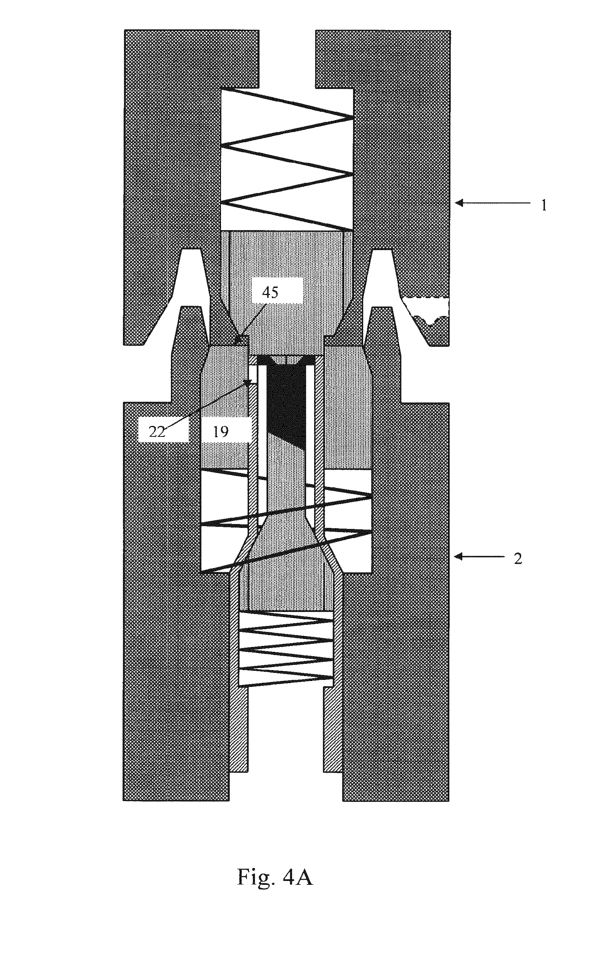

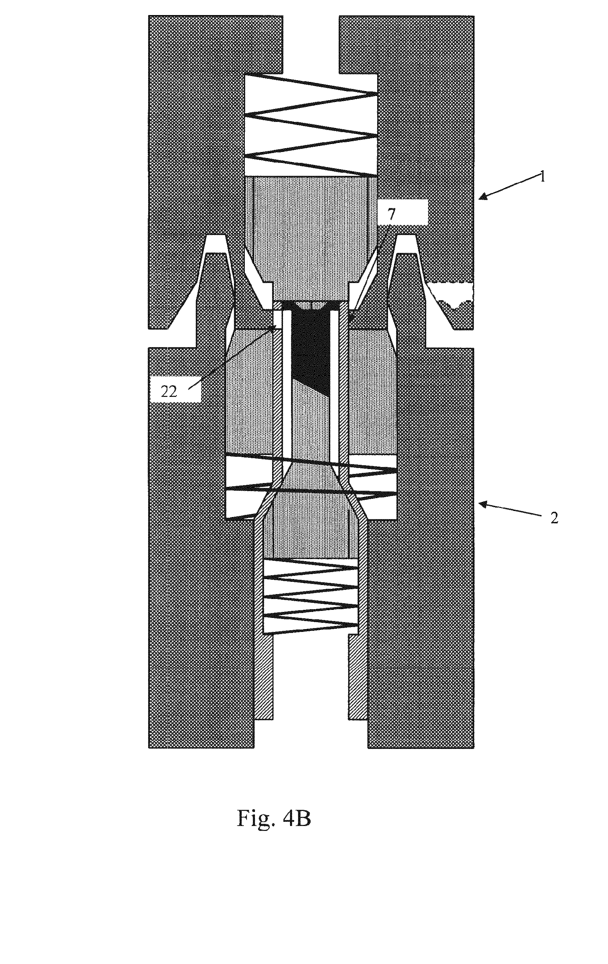

Figs. 4A and 4B show two intermediate situations during coupling and decoupling, where 4B shows the situation where the needle openings (22) are closed by the first opening (7) (optionally a second sealing element). Fig. 4A shows the situation where the first (1) and second (2) couplers are just in contact, either to be coupled or decoupled. In all situations from the couplers (1) and (2) being in contact, as seen in Fig. 4B, to being fully coupled as seen in Fig. 3, the front surface(s) part (45) is at any time in full contact with the front surface of the second valve body (19). Therefore, between these situations there will never be any fluid containing space in contact to the externals. The coupling is therefore a non-drip fluid coupling. To further tighten the coupling, the first (7) and / or second (17) openings may comprise sealing elements in the sides of the openings.

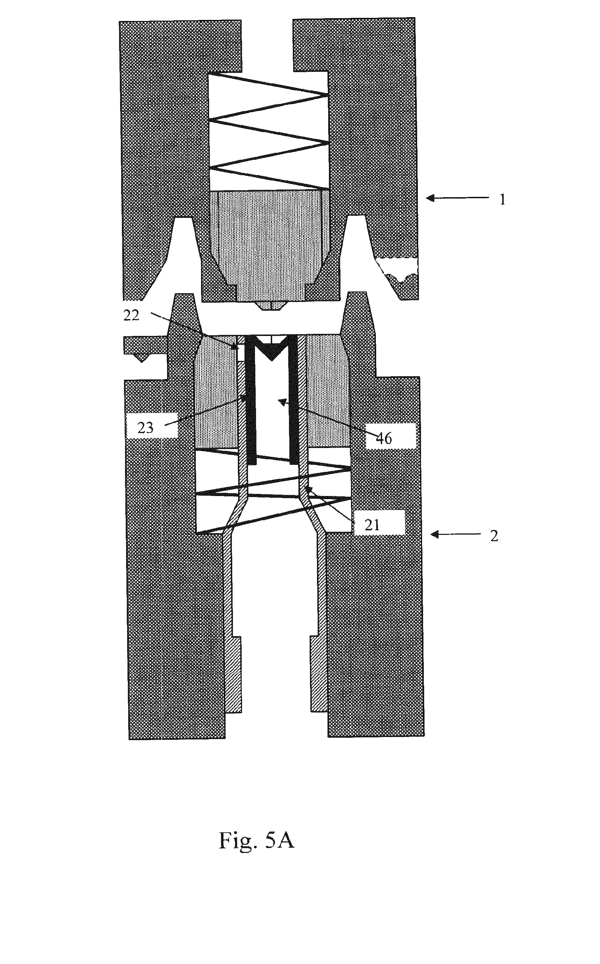

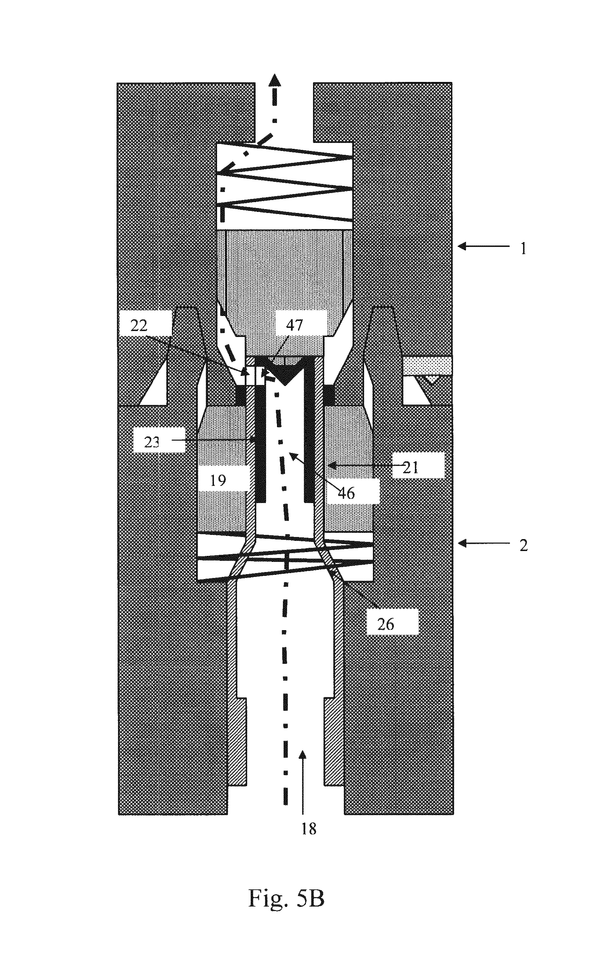

Figs. 5A and 5B show an alternative and slightly simpler embodiment, where the hollow needle (21) does not comprise a third valve body (24), only the sealing element (23). In this embodiment at least the second valve body (19) forms a seal of the needle openings (22), but in a more advanced embodiment the sealing element (23) itself has an inner flow channel (46) and sealing element opening(s) (47). The outer surface of the sealing element (23) in this embodiment at least a part of the external surface of sealing element (23) forms a seal against the internal wall of the hollow needle (21), thus confines the flow to the inner flow channel (46). When not coupled the sealing element (23) is in a rotational position where the sealing element opening(s) (47) is not in contact with the needle opening(s) (22), thus also sealing the needle opening(s) (22) from the inside, however, when couplers (1) and (2) are coupled, as seen in Fig. 5, the rotation of the sealing element (23) has rotated the sealing opening(s) (47) to communication with the needle opening(s) (22). During decoupling the sealing element (23) is rotated back again, re-sealing the needle opening(s) (22) from the inside.

Since this embodiment has no valve element (24) pressing against a narrowing (26) in the second fluid coupling (2), a substantial pressure of fluid from the second fluid communication (18) may, due to softness of the materials of the sealing member (27) and the second valve body (24), risk to be squeezed out of the system. Therefore this embodiment is most suited for systems where the pressure from the fluid is limited.

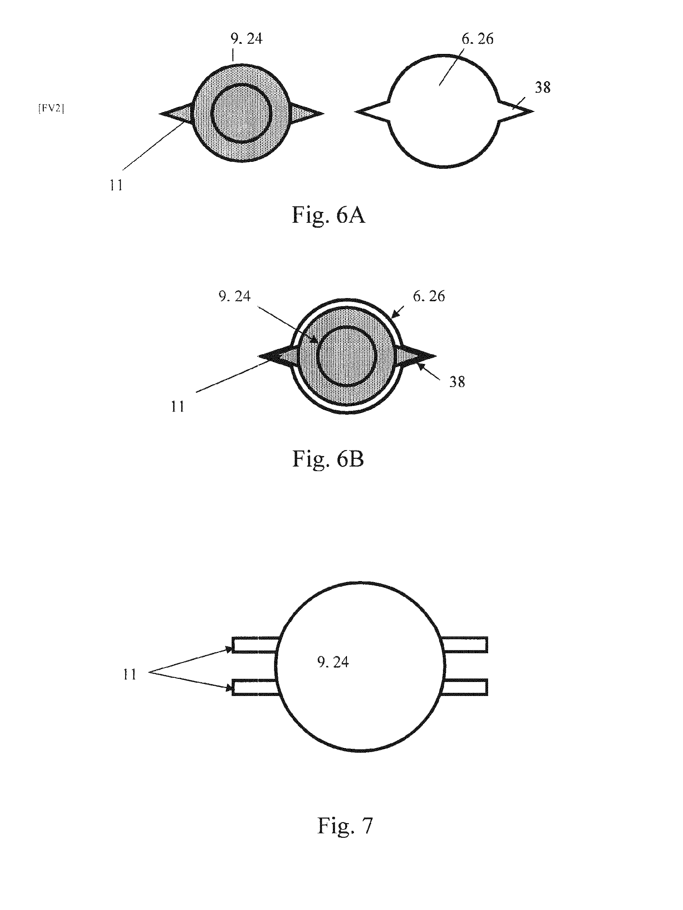

Fig. 6A shows a top view of one of the valve bodies (9) and (24) with slide extensions (11) and one of the first (6) or second (26) chambers having recesses (38) matching the slide extensions (11). Fig. 6B shows the valve body (9) or (24) inside the first (6) or second (26) chamber, where during movement of the valve bodies (9) and (24), the slide extensions (11) slide in the recesses (38), thus preventing the valve bodies (9) and (24) from rotation. The valve bodies (9) and (24) may be equipped with any number and configuration of such slide extensions (11), and Fig. 7 shows an embodiment with two sets of parallel slide extensions (11).

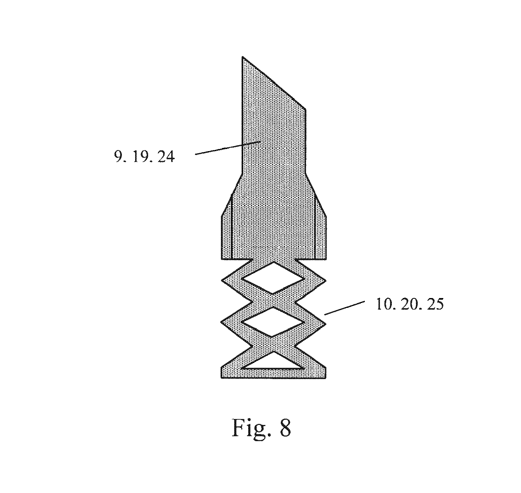

Fig. 8 shows how the valve bodies (9), (19) and (24) and their respective biasing members (10), (20) and (25) are formed as one single body.

The two couplers (1) and (2) preferably comprise attaching means for connecting the couplers securely to external fluid systems, such as e.g. one of the couplers (1) or (2) to be coupled to a catheter forming a fluid connection to the first fluid communication (8), and the other of the couplers (2) or (1) to be coupled to the urine bag forming a fluid connection to the second fluid communication (18), or vice versa.

It is just as essential that these connections do not detach un-intently as for the coupling of the first (1) and second (2) couplers, and they therefore comprise means forming such an attachment. This could include forming a permanent attachment by adhering, welding etc., any such means as known in the art, or detachable means such as windings, clamping means etc., any such means as known in the art.

## Claims

1. Non-drip fluid coupler comprising a first coupler and a second coupler, where
- the first coupler has first chamber connected to a first fluid communication and an first opening, a first valve body being biased against the first opening by a first biasing member,
- the second coupler has a second chamber connected to a second fluid communication and a second opening, a second valve body being biased against the second opening by a second biasing member,
- a hollow needle element is positioned or formed within the second chamber and comprises a sealing element,
wherein the first and second coupler comprises guiding means adapted to aid the connection of the first and second couplers.

2. Non-drip fluid coupler according to claim 1, where the guiding means comprises
- first deepening(s) of one of the couplers, where the cross section area of the first deepening(s) being substantially funnel shaped having an outer section and an inner section, where the outer width of the outer section is substantially larger than any width of the inner section, and
- first protruding structure(s) with a shape fitting into the inner section(s).

3. Fluid coupler according to claim 1, wherein the outer width of the outer section is at least 1.2 times larger than any width of the inner section.

4. Fluid coupler according to claim 3, wherein the outer width of the outer section is at least two times larger than any width of the inner section.

5. The fluid coupler according to one of claims 1-4, wherein the first and second coupler comprise interlocking means preventing them from any movement in a longitudinal direction when coupled.

6. The fluid coupler according to claim 5, wherein the interlocking means comprises rotation guiding means assisting to couple them by rotation.

7. The fluid coupler according to claim 6, wherein the rotation guiding means comprises at least one 'receiving' shape of one of the couplers and at least one wing of the other coupler adapted to fit into the 'receiving' shape(s) the wing(s) being introduced into the receiving shape(s) by rotating the couplers relative to each other.

8. The fluid coupler according to any of the preceding claims, wherein the combined first protruding structure(s) is rotationally symmetric.

9. The non-drip fluid connector according to any preceding claims, where the hollow needle element comprises at least one needle opening being sealed by the second valve body when the first and second couplers are not coupled.

10. The non-drip fluid connector of any preceding claim, where one of the first and second valve bodies has second protrusions matching a second deepening of the other of the first and second valve bodies, and where the second protrusion and the second deepening are shaped such that when mated they are interlocked in a rotational direction.

11. The non-drip fluid connector of any of the preceding claims, where a third valve body is introduced to be biased against a narrowing of the hollow needle element and the sealing element by a third biasing member, and where the contact surfaces between the third valve body and the sealing element are such that a rotation of the sealing element moves the third valve body away from the sealing element.

12. The non-drip fluid connector of claim 11, where the contact surfaces are such that they have an angle different to being orthogonal to a longitudinal axis relative the extension of the alignment line of the third valve body and the sealing element.

13. The non-drip fluid connector of one of claims 11 or 12 where the first valve body and the third valve body are prevented from rotating within the first chamber and hollow needle, respectively.

14. The non-drip fluid connector of any of the preceding claims, wherein the first opening has a narrowing matching the shape of the first valve body in such a manner that, when the first valve body is in the closed position, the first opening is sealed from any fluid communication through the first opening.

15. The non-drip fluid connector of any of the preceding claims, wherein the first and second couplers comprise attaching means to attach external fluid systems to each coupler forming fluid connection to the first and second fluid communications respectively, and adapted to keep the external fluid systems firmly fixed to the first and second couplers, and where the attaching means comprises adhesives, windings, threads, a clamp device and / or a pin to be positioned into a notch during coupling.
